# EUROPEAN PATENT APPLICATION

(11) **EP 4 751 637 A1**
(43) Date of publication of application: **03.06.2026**
(21) Application number: 24216947.2
(22) Date of filing: 02.12.2024
(51) Int. Cl.: A61B 5/00, G06T 7/00

(54) **SKIN MONITORING DEVICE AND METHOD**

(71) Applicant: Dermatoo, 4053 Chaudfontaine (BE)
(72) Inventor: DELARBRE, Julien, 4053 Embourg (BE); DELARBRE, François, 4219 Ambresin (BE); DOYEN, Sébastien, 1200 Woluwe-Saint-Lambert (BE)
(74) Representative: Calysta NV

(57) **Abstract**

Computer-implemented method for monitoring skin changes over time of the skin of a subject, the method comprising: capturing a guidance reference image comprising a portion of the skin; capturing a first image of a set of monitoring images comprising the portion of the skin and a calibration reference adhered to the skin; calculating a similarity metric between the guidance reference image and the first image; wherein, during the capturing of subsequent images of the set, the guidance reference image is displayed as an overlay based on the similarity metric thereby providing guidance to a user during capturing.

## Description

### Technical Field

The present invention relates to the technical field of devices and computer-implemented methods for image-based monitoring of the skin of a subject.

### Prior art

Skin monitoring is crucial in various medical conditions, such as wounds, burns, and ulcers, or cosmetic concerns, such as pigmentation changes, texture irregularities.

Traditional skin monitoring methods, such as use of swab, use of rulers, or acetate tracing, are heavily user dependent, and often involve physical contact, which should be avoided for hygienic reasons.

Imaging techniques, such as photography, have gained increasing attention as they offer a contactless, non-invasive approach to assess the skin, thereby reducing the risk of contamination. However, existing image-based approaches have several deficiencies.

A major drawback of current image-based approaches for skin monitoring is the lack of reproducibility and standardization, which can lead to errors like misclassification or misdiagnosis, which may delay or compromise clinical interventions.

Lack of reproducibility can arise from variability in image capture conditions such as lighting, camera angle, and distance from the skin.

Furthermore, the subsequent measurements performed on these images often rely on subjective, heavily user-dependent techniques, such as rulers for wound length and width assessment, or the ABCDE method (Asymmetry, Border, Color, Diameter, Evolving) for assessing potential malignancy of moles.

This problem is even more pronounced in non-clinical settings, such as homes, nurseries, or elder care facilities, where imaging is often performed by different, untrained individuals using their own mobile devices. This introduces additional variability from differences in operators' techniques and devices.

Another drawback of current image-based skin monitoring approaches is the limited information that can be derived therefrom. Typically, these solutions attempt to obtain approximate measurements, such as dimensions, or qualitative information, but fail to capture comprehensive data indicative of skin processes.

Therefore, existing image-based skin monitoring approaches are inadequate for ensuring accurate monitoring, especially when images are captured over extended periods of time.

Accurate image-based skin monitoring is highly desirable to draw meaningful conclusions and generate quantitative data, which is crucial for skin conditions such as chronic wounds.

Indeed, chronic wounds that are inadequately treated are at a higher risk of infection, which can lead to severe negative consequences on mobility and quality, particularly in the elderly and individuals with underlying conditions such as diabetes.

The preceding discussion of the prior art is intended to facilitate an understanding of the present invention only. The discussion is not an acknowledgement or admission that any of the material referred to is or was part of the common general knowledge as at the priority date of the application.

### Brief summary of the invention

It is an object of the invention to overcome the disadvantages of the prior art, or at least provide an alternative to the prior art. It is in particular an object of the invention to enable accurate and consistent skin monitoring using imaging techniques.

According to the invention, this object is solved by a computer-implemented method according to claim 1 for monitoring skin changes overtime of the skin of a subject, the method comprising the steps of:
- capturing a guidance reference image comprising a portion of the skin of the subject;
- capturing a first image of a set of monitoring images comprising the portion of the skin of the subject and further comprising a calibration reference adhered to the skin;
- calculating a similarity metric between the guidance reference image and the first image of the set;
wherein, during the capturing of subsequent images of the set of monitoring images, the guidance reference image is displayed as an overlay based on the similarity metric thereby providing guidance to a user during capturing.

In the context of the invention, the term "capturing" refers to the act of obtaining an image or other visual data through the use of a device that is equipped for this purpose. Such a device may include, but is not limited to, a mobile phone, a digital camera, or any other apparatus capable of performing the function of acquiring an image. Additionally, the device is further configured to process the captured image. This processing step may include, for example, enhancing the image quality, extracting relevant features, or performing analysis to prepare the image for subsequent steps as described in the invention.

A "captured image" may be a 2D (two-dimensional) image or 3D (three-dimensional) image.

2D images are captured using a device equipped with a 2D imaging sensor, e.g. a visible-light image sensor. The 2D image may be a single channel 2D image, e.g. a 2D grayscale image, or a multi-channel 2D image, e.g., a RGB 2D image.

3D images can be captured using a 3D imaging sensor, e.g. a depth sensor (such as a Time-of-Flight sensor, a LiDAR), or by capturing a plurality of 2D images with a 2D imaging sensor and processing them using a photogrammetry-based technique, to reconstruct a 3D image therefrom. The 3D image may be a single-channel 3D image, e.g. a depth map, or a multi-channel 3D image, such as an RGB-D image.

The captured image may also be a frame of a video stream.

A "video stream" is a sequence of images (frames) captured sequentially over time by an image-capturing device. The frames are typically captured at a capture frequency and displayed at a display frequency, which are measured in frames per second (fps). For example, the video stream may be a live video stream wherein the sequence of frames is captured and displayed by the image-capturing device in real time to provide a dynamic preview to the user, before capture of a still image.

The "user" is the individual who captures the first image of the set. The guidance reference image may also be captured by the user, or it may be captured by another individual. The user may be the subject, who takes a photo of themselves without external assistance, or another individual, such as a healthcare professional (e.g., a nurse or physician), a caregiver, a family member assisting the subject.

The "subject" is the individual whose portion of skin is being imaged.

In the first step, a guidance reference image comprising a portion of the skin of the subject is captured. This "guidance reference image" comprises a portion of the subject's skin that is intended for observation over time, for example a portion including a skin alteration or lesion (e.g., a wound, an ulcer, a naevus, a scar, a burn) or any skin feature likely to change over time. This guidance reference image serves as a reference for alignment of the image capture device during capture of the subsequent images.

In the second step, a first image of a set of monitoring images comprising the portion of the skin of the subject and further comprising a calibration reference adhered to the skin is captured.

The guidance reference image, the first image and the subsequent images of the set may be captured by the same imaging sensor or by different imaging sensors.

For example, the guidance reference image may be an image captured by a first image-capturing device operated by clinical personnel e.g. during a dermatological assessment, and subsequently transmitted to a second image-capturing device, e.g. a smartphone owned by the subject. In this case, the first image may be captured by the subject, using the second image-capturing device in a non-clinical setting, e.g. at home.

This "first image" is the earliest chronologically captured image in the set of monitoring images. This first image serves to calibrate the guidance reference image, and to ensure consistent capture conditions between the guidance reference image and subsequent monitoring images.

Thus, this first image comprises, or at least partially overlaps with, the same portion of the subject's skin in the guidance reference image. However, the first image additionally comprises a calibration reference adhered to the skin. The calibration reference is not necessarily present in the guidance reference image. For example, the guidance reference image may be captured immediately following an injury, while the first image may be captured in a follow up phase, after adhering the calibration reference.

The "calibration reference" is an object arranged within the field of view of the first image comprising standard, or at least known, visual information (like color, brightness, dimensions, and the like) which can be used to correct images and compensate for variations in capture conditions across different images.

For example, the calibration reference may comprise a geometric reference for spatial calibration and/or color reference for color calibration.

A "geometric reference" is a calibration reference having known geometrical information that is used to determine geometric transformation data (e.g., a rotation or distortion matrix) between the guidance reference image and the first image e.g. to compensate distortions caused by differences in the orientation of the imaging device relative to the subject's skin.

A "color reference" is a calibration reference having known color information that is used to determine color transformation data (e.g., a color correction matrix comprising components like hue values, chrominance values, luminance values, or a combination thereof) between the guidance reference image and the first image e.g. to compensate for different environmental lighting conditions.

When a "color correction matrix" is used, this matrix may enable adjustments for white balance, gamut mapping between color spaces (e.g., RGB to sRGB and/or to CIE LAB), non-linear transformations like gamma correction, spectral sensitivity alignment, noise and chromatic aberration reduction, uniformity correction for sensor inconsistencies, and/or dynamic adaptation to varying lighting conditions, typically calibrated using reference targets or environmental sampling.

For example, the geometric reference may comprise, according to an embodiment, a one-dimensional marker (e.g., a barcode, a ruler), a two-dimensional marker (e.g., an Aruco marker, a two-dimensional barcode, a QR code), or a board of two-dimensional markers (e.g., an Aruco board).

For example, the color reference may comprise one or more of the group of a red marker, a yellow marker, a brown marker, a black marker, a grey marker.

In embodiments, the calibration reference comprises at least one color reference and at least one geometric reference. For example, the calibration reference may comprise a plurality of color references and geometric references arranged in a grid pattern.

In the third step, a similarity metric is calculated between the guidance reference image and the first image.

When the first image is a frame of a video stream, the similarity metric can be further calculated for the other frames of the video stream. For example, the similarity metric may be calculated in real-time for each frame of a live image preview displayed on a screen of the image capturing device.

The "similarity metric" is indicative of the degree of similarity between the guidance reference image and the first image.

For example, the similarity metric may be calculated based on a correlation (e.g. a cross-correlation matrix, a peak cross-correlation, a normalized cross-correlation), a structural similarity index, an average squared difference, a signal-to-noise ratio (SNR)-based metric (e.g. a peak SNR, or PSNR), one or more matched features (e.g., a vector of matched features obtained using a feature matching algorithm, e.g. a vector of locations of key-points in the two images with their corresponding descriptors), a distance (e.g. a Procrustes distance, a Hausdorff distance), or any combination thereof.

For example, the similarity metric may be calculated using a feature matching algorithm.

Feature matching algorithms typically comprise a feature detection stage (optionally, preceded by an image segmentation stage), a feature extraction stage and a feature matching stage.

The "feature detection" stage allows to detect regions of interest (e.g. points) in each of the first image, and the guidance reference image.

The "feature extraction" stage allows to extract descriptors from the detected regions of interests.

The "feature matching" stage allows to match the features extracted from the guidance reference image and the features extracted from the first image, based on their respective descriptors.

When the similarity metric is calculated using a feature matching algorithm, any one of the image segmentation (if present), feature detection, feature extraction, or feature matching stages may be implemented by a machine learning or deep learning model.

In the fourth step, the guidance reference image is displayed as an overlay during the capturing of subsequent images of the set of monitoring images, wherein the overlay is based on the similarity metric.

By displaying the guidance reference image "as an overlay" based on the similarity metric, it is meant that the overlaid guidance reference image is superposed on, or blended with, the subsequent image, while allowing portions of the subsequent image to remain at least partially visible. Thus, during capturing, information is provided to the user about how closely the current capture conditions match the intended capture conditions.

In preferred embodiments, the overlay comprises applying a transparency coefficient to the guidance reference image and/or generating a mask of the guidance reference image.

In this case, the guidance reference image may be displayed as a semi-transparent layer and/or a mask superposed on, or blended with, a subsequent monitoring image, e.g. in a live image preview of the subsequent monitoring image.

Alternatively, the overlay comprises guidance to the user in the form of visual indicia based on the similarity metric. The visual indicia comprises, for example, arrows, or green/light indicator.

The method facilitates real-time alignment of the portion of the skin being captured relative to its position and orientation in the guidance reference image. This ensures consistency in the image capture conditions over time.

Therefore, the method is particularly advantageous for remote skin monitoring, which often takes place in different environments (e.g. clinical and non-clinical settings) and relies on different users for capturing the images over time (e.g. different caregivers assisting the subject, or the subjects themselves), often using different image-capturing devices (e.g., each caregiver using their personal mobile device).

Moreover, by combining consistent image capture conditions over time with the presence of the calibration reference in the monitoring images, it is possible to accurately derive from the set and/or from the guidance reference image, quantitative information about the status of the subject's skin, and detect if changes occur over time.

When the guidance reference image is captured according to standardized protocols or rules, the method further facilitates inter-subject standardization. Inter-subject standardization is valuable for creating training datasets for predictive model, such as predictive models of wound healing, or skin treatment outcome.

Overall, the method allows to achieve a more accurate monitoring of a portion of the skin of the subject.

Optionally, the method comprises a step of selecting, from a plurality of images, an image to be used as guidance reference image.

For example, the method may comprise displaying thumbnails or previews of a plurality images to the user, and receiving a user input corresponding to the selection of a specific image of the plurality as the guidance reference image.

The guidance reference image, the first image and/or any subsequent image of the set may be a 3D image reconstructed based on 2D images captured by a 2D imaging sensor.

In this case, according to an embodiment, the method comprises:
- capturing at least two, preferably at least three, more preferably at least five, 2D images of the portion of skin of the subject, the 2D images being captured under different angles; and
- reconstructing a 3D image of the portion of skin based on the 2D images using a photogrammetry-based technique.

For example, the 3D image may be reconstructed using a SFM (structure from motion) algorithm.

In embodiments, the method comprises a step of segmenting the guidance reference image, the first image, and/or the subsequent images of the of the set.

The similarity metric is calculated between the guidance reference image and the first image. When the calculation of the similarity metric is preceded by an image segmentation step, the similarity metric may be calculated between the segmented guidance reference image and segmented first image.

In embodiments, the method comprises:
- isolating, in the first image, a region of interest comprising the skin portion from the background, and
- isolating, in the first image, a region of interest comprising a skin alteration or a calibration reference from the skin portion.

In this case, the similarity metrics can be calculated between the guidance reference image and the first image based on the pixels of the first image that lie within the region of interest comprising the skin portion, but outside the region of interest comprising the skin alteration and/or the calibration reference.

In embodiments, the similarity metric is calculated using a feature matching algorithm.

Examples of algorithms for feature detection and/or feature extraction are: SIFT (Scale-Invariant Feature Transform), SURF (Speeded-Up Robust Features), ORB (Oriented FAST and Rotated BRIEF).

Examples of algorithms suitable for feature matching are FLANN (Fast Library for Approximate Nearest Neighbors), RANSAC (Random Sample Consensus) or Brute Force Matcher.

When a feature matching algorithm is used, the similarity metric may be the output of the feature matching stage.

Alternatively, the similarity metric is calculated using a contour matching algorithm.

In embodiments, the similarity metric is calculated using machine learning or deep learning algorithm.

However, the similarity metric may also be calculated using a mathematical or statistical algorithm or a hybrid algorithm (e.g., a combination of a rule-based feature detection, such as SIFT, combined with a ML-based feature extraction or matching).

When the first image is a frame of a video stream, the calculation of the similarity metric between the guidance reference image and the first image may be repeated for a plurality of frames of the video stream. This repetition may be at a fixed frequency (e.g. for each frame of the video stream, or at intervals corresponding to every N frames, where N is a positive integer) or at a variable frequency (e.g., the user manually selects specific frames from the video stream via an input interface, such as a touchscreen where the live video stream is displayed, and the calculation is implemented for each selected frame).

In embodiments, the overlay of the guidance reference image comprises applying a transparency coefficient to the guidance reference image.

This allows to obtain a semi-transparent foreground image from the guidance reference image, which can be blended with a monitoring image as a background image.

For example, the method may comprise applying a global transparency coefficient to all pixels of the guidance reference image to obtain the semi-transparent foreground image.

However, it is also possible to apply different transparency coefficients to different pixels of the guidance reference image, to have pixel-wise modulation of transparency.

For example, the guidance reference image may be multiplied by an alpha channel matrix where each element of the alpha channel matrix has a value comprised between 0 (corresponding to fully transparent) and 1 (corresponding to fully opaque).

The transparency coefficient may be modified by the user, for example via a slider of a graphical user interface (GUI).

In embodiments, the overlay of the guidance reference image comprises generating a mask of the guidance reference image using an edge detection algorithm.

For example, the overlay may comprise generating a mask from the guidance reference image by applying a segmentation and/or edge detection algorithm to the guidance reference image.

For example, the method may comprise detecting the edges corresponding to boundaries between the portion of the skin (e.g. a limb of the subject) and the background, and generating a mask, wherein a maximum opacity value is assigned to pixels of the mask corresponding to a detected edge, and a minimum opacity value is assigned to pixels of the mask not corresponding to a detected edge (e.g., corresponding to background, or to a central part of the portion).

In embodiments, the overlay of the guidance reference image comprises visual indicia.

For example, visual indicia may be arrows or text for indicating to the user a direction toward which the image capture device should be moved or rotated.

Visual indicia may be generated based on data received from a sensor configured to sense positional data of the image capture device, e.g. an accelerometer, or a gyroscope.

When the first image (or a subsequent image) is a frame of a video stream, the displaying of the overlay during capture may be repeated for a plurality of frames of the video stream. This repetition may be at a fixed frequency (e.g. for each frame of the video stream, or at intervals corresponding to every N frames, where N is a positive integer) or at a variable frequency (e.g., the user manually selects specific frames from the video stream via an input interface, such as a touchscreen where the live video stream is displayed, and the displaying step is implemented for each selected frame).

In embodiments, the method further comprises a step of calibrating the guidance reference image based on the calibration reference.

In embodiments, the calibration reference comprises a geometric reference.

This allows to perform a geometric calibration so as to accurately measure geometric parameters of the portion of the skin in the calibrated guidance reference image and/or in the calibrated subsequent images of the set. This is particularly advantageous to monitor skin processes associated with geometrical changes, such as the changes of the width or length of a wound during healing.

In embodiments, the geometric reference comprises a one-dimensional marker.

For example, the geometric reference may comprise a one-dimensional barcode, a ruler.

In embodiments, the geometric reference comprises a two-dimensional marker.

For example, the geometric reference may comprise an Aruco marker, a two-dimensional barcode, a QR code. The geometric reference may comprise a board of two-dimensional markers, e.g., an Aruco board, a chessboard, ora board comprising a combination of two-dimensional markers.

In embodiments, the calibration reference comprises a color reference.

This allows to perform a color calibration so as to accurately measure color parameters of the portion of the skin in the calibrated guidance reference image and/or in the calibrated subsequent images of the set. This is particularly advantageous to monitor skin processes associated with a change of color, such as the appearance of a black color during tissue necrosis.

In embodiments, the geometric reference (e.g. one or more one-dimensional markers or two-dimensional markers of the calibration reference) is colored.

In this case, the colored geometric reference can be used for both color calibration and geometric calibration.

The calibration step may be preceded by a conversion step for converting the guidance reference image and/or the first image into a different color space for subsequent calibration (e.g. RGB, CIE L*a*b*, CIE L*U*V*, YUV, YcbCr, YES, HSV).

In embodiments, the color reference comprises one or more of the group of a red marker, a yellow marker, a brown marker, a black marker, a grey marker.

These color markers are particularly advantageous to monitor different skin tissue types, such as (healthy) epithelial tissue, necrotic tissue, granulation tissue, fibrous tissue, slough, eschar, as well as fluid such as blood or serous exudate.

This allows to monitor changes in skin tissue over time, such as a transition from necrotic tissue to granulation tissue and epithelial tissue, and/or to track the presence of fluids like blood or serous exudate.

In embodiments, the calibration reference comprises at least one color reference and at least one geometric reference.

This allows to simultaneously measure geometric parameters and color parameters of the portion of the skin, thereby ensuring a more comprehensive monitoring of the skin. For example, it is possible to monitor whether a color shift (e.g. a shift towards pink at the rims of a wound, which may be due to new epithelial tissue formation) is accompanied by a geometric change (e.g. a reduction of the distance the rims of the wound in the previous example).

In embodiments, the at least one color reference and the at least one geometric reference are arranged in a grid pattern. For example, the calibration reference may comprise a plurality of two-dimensional markers and color markers arranged to form a board e.g. as in a checkerboard.

In embodiments, the method further comprises the steps of:
- segmenting the set of monitoring images thereby isolating a region of interest comprising a skin alteration from a background;
- classifying pixels of the isolated region of interest into a class of a set of predefined tissue classes based on a color of the pixel;
wherein the predefined tissue classes comprise at least one of: fibrous tissue, necrotic tissue, blood, exudate or epithelial tissue.

This is particularly advantageous for monitoring changes over time of the skin which are linked to biological processes, such as wound healing.

In embodiments, the region of interest comprises a skin lesion, the skin lesion being one of the group of: a wound; a scar; an ulcer; and/or a burn.

According to a second aspect, the invention further relates to a skin monitoring device for monitoring skin changes over time of the skin of a subject comprising means configured to perform the method according to any of the embodiments herein described.

The device is embodied as a set of physical parts or modules, which may housed within a same machine (e.g., in a smartphone, a laptop, or a tablet) or in different, e.g. remote, machines. The device may e.g. have functions distributed over a cloud infrastructure and be available to users as a cloud-based service.

The device comprises an image capture module configured to capture the guidance reference image and/or the first image and/or the subsequent images of the set of monitoring images.

The image capture module may comprise a 2D imaging sensor, e.g. a visible-light camera, for capturing two-dimensional images and/or a 3D imaging sensor, e.g. a Time-of-Flight sensor, for capturing three-dimensional images.

The device comprises a processor configured to calculate the similarity metrics.

The term "processor" should not be construed to be restricted to hardware capable of executing software, and refers in a general way to a processing device, which can for example include a computer, a microprocessor, an integrated circuit, or a programmable logic device (PLD). The processor may also encompass one or more Graphics Processing Units (GPU), whether exploited for computer graphics and image processing or other functions. Additionally, the instructions and/or data enabling to perform associated and/or resulting functionalities may be stored on any processor-readable medium such as, e.g., an integrated circuit, a hard disk, a CD (Compact Disc), an optical disc such as a DVD (Digital Versatile Disc), a RAM (Random-Access Memory) or a ROM (Read-Only Memory). Instructions may be notably stored in hardware, software, firmware or in any combination thereof.

The device comprises a display configured to display the guidance reference image as an overlay based on the similarity metric. The display may further be configured to display thumbnails or previews of images stored in a memory.

In embodiments, the device comprises a memory such as a local nonvolatile storage (e.g., as flash memory), or a remote storage accessible via a network (e.g. a cloud-based storage)

The memory may be configured to store the guidance reference image and/or one or more images of the set of monitoring images.

In embodiments, the device comprises an input module such as a touchscreen, a touchpad, a physical button, a keyboard, a mouse, or a microphone.

The input module may be configured to receive a user input (e.g. via a touchscreen), corresponding to the selection of an image to be used as guidance reference image and/or as first image.

In embodiments, the device comprises an accelerometer and/or a gyroscope.

According to a third aspect, the present invention also relates to a computer readable storage medium comprising instructions which, when executed by a computer, cause the computer to carry out the steps of the method according to any of embodiments herein described.

According to a fourth aspect, the present invention also relates to a computer program for monitoring skin changes over time of the skin of a subject, the computer program comprising program code instructions which, when executed by a computer, cause the computer to carry out the method according to any one of the embodiments described hereabove.

Other embodiments according to the present invention are mentioned in the appended claims and the subsequent description of an embodiment of the invention.

### Brief description of the Drawings

Exemplary embodiments of the invention are described using the figures. It is to be understood that these figures merely serve as examples of how the invention can be implemented and are in no way intended to be construed as limiting for the scope of the invention and the claims. Like features are indicated by like reference numerals along the figures. In the figures:
**Fig.** 1 is a flowchart schematically illustrating the steps of a method for monitoring skin changes over time of the skin of a subject;
**Fig. 2A** schematically illustrates a step of capturing a guidance reference image;
**Fig. 2B** schematically illustrates a step of capturing a first image;
**Fig. 3A** schematically illustrates a guidance reference image;
**Fig. 3B** schematically illustrates a first image;
**Fig. 3C** schematically illustrates a subsequent image where a guidance reference is displayed as an overlay;
**Fig. 4** schematically illustrates an exemplary calibration reference.

### Detailed description of an embodiment of the invention

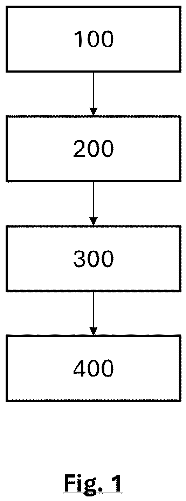

**Fig. 1** is a schematically illustrated the steps of a method for monitoring skin changes over time of the skin of a subject according to the present invention. The method comprises a first step 100 of capturing a guidance reference image A comprising a portion of the skin of the subject; a second step 200 of capturing a first image B of a set of monitoring images, the first image B comprising the portion of the skin of the subject and a calibration reference adhered on the skin; a third step 300 of calculating a similarity metric between the guidance reference image A and the first image B captured in the previous capturing steps 100, 200; and a fourth step 400 of capturing a subsequent image of the set of monitoring images.

During the fourth step 400, the guidance reference image A is displayed as an overlay based on the similarity metric calculated in the previous calculation step 300.

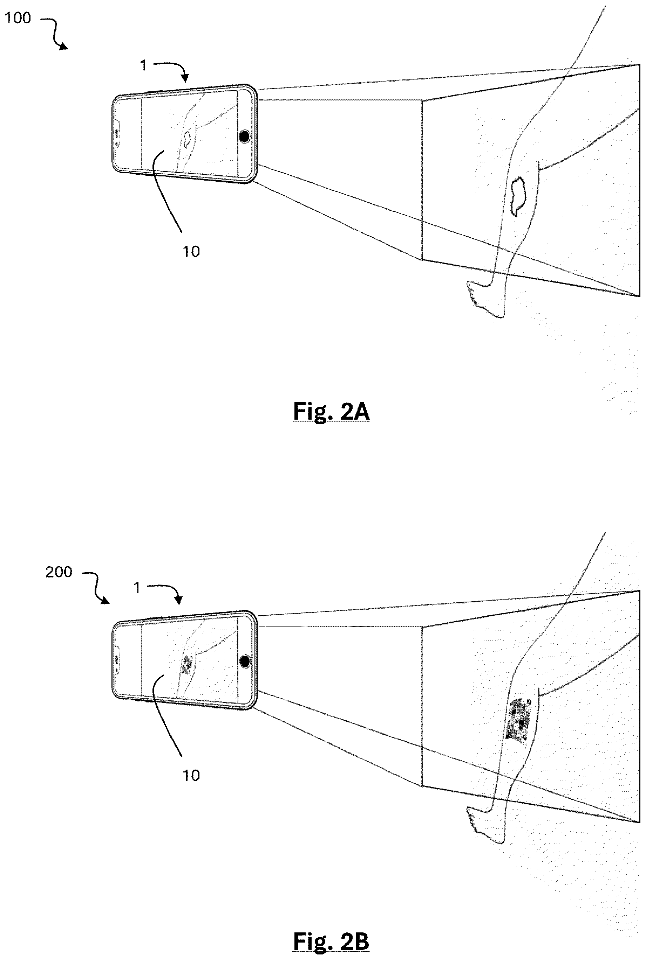

**Fig. 2A** is a schematic representation of a first capturing step 100 in which a guidance reference image A comprising a portion of the skin of the subject is captured. In this example, the portion of the skin of the subject is a portion of a lower limb.

The guidance reference image A may be captured by a user who can be e.g. a nurse, a physician, an esthetician, a caregiver, a family member of the subject. For example, the guidance reference image A may be captured immediately following an injury, e.g. by emergency personnel, for documenting the presence and extent of a skin lesion resulting from the injury. The guidance reference image A may also be captured to document a preexisting skin alteration, for example as part of a decision to initiate or modify a medical or esthetic treatment.

In fig. 2A, a skin alteration 3 is schematically represented as an irregular shape. The skin alteration 3 may be a skin lesion, such as a wound, a scar, an ulcer, and/or a burn.

In the example of fig. 2A, the guidance reference image A is captured using a device 1 positioned laterally relative to the subject, so that the skin alteration 3 is located within the field of view (FOV) of an imaging sensor (e.g. a visible-light image sensor, not shown in fig. 2A) of the device 1.

The guidance reference image A may be captured by a device 1, which may be a smartphone as depicted in fig. 2A, or any device 1 suitable for capturing an image (e.g. digital camera, a laptop, a tablet). The device 1 comprises a screen where a live image preview can be displayed during capturing. In this case, the guidance reference image A may be a frame of the live image preview.

The guidance reference image A may be processed, for instance, by enhancing image quality, extracting relevant features (e.g. using a deep learning-based feature matching algorithm), performing edge detection (e.g., for detecting the boundaries between the portion of the subject's skin and the background and/or the boundary of the skin alteration), segmentation, color space conversion, or applying other image analysis techniques.

**Fig. 2B** is a schematic representation of a second capturing step 200, in which a first image B is captured.

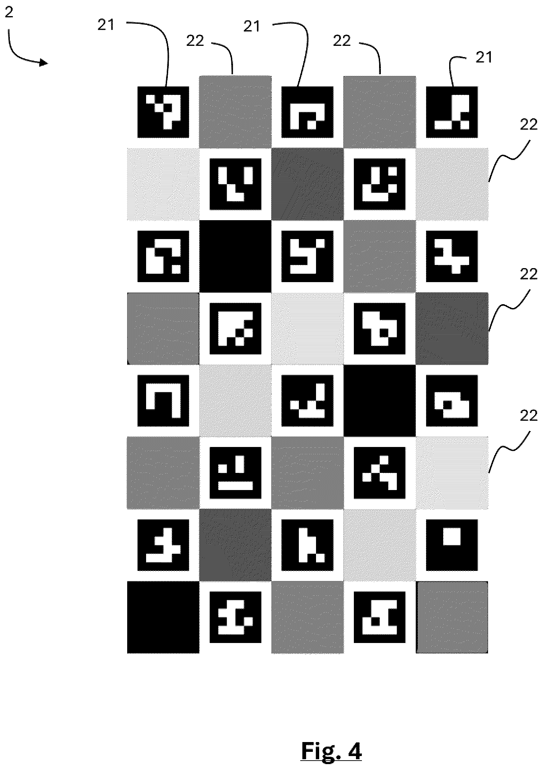

The first image B is captured to image the same portion of the subject's skin (in this case, a lower limb) which is in the guidance reference image A, or at least a portion of the subject's skin that partially overlaps with the portion that is in in the guidance reference image A. The first image B is captured after a calibration reference 2 is adhered to the portion. In the example of fig. 2B, the calibration reference 2 is adhered to the lower leg of the subject. This calibration reference 2 comprises a plurality of markers arranged in a grid pattern, as in a checkerboard. For example, the calibration reference 2 may comprise a plurality of geometric markers and/or color markers arranged in a rectangular board. However, different arrangements and types of markers may be used. The calibration reference 2 will be described in more detail with reference to fig. 4.

In the capturing step 200 of fig. 2B, the device 1 is also positioned laterally with respect to the subject. The second capturing step 200 may be performed by the same user who captures the guidance reference image A, or a different user.

The first image B may be processed using an image analysis technique. For example, the first image B may be segmented e.g. to isolate the calibration reference 2. The first image B may be processed using the same image analysis technique applied to the guidance reference image A, or a different technique. The first image B may also be used to calibrate the guidance reference image A.

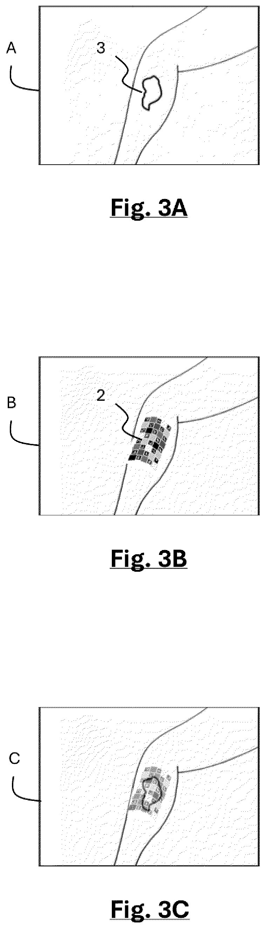

**Fig. 3A** is a schematic representation of the guidance reference image A captured in the capturing step of fig. 2A. This guidance reference image A comprises the portion of a lower limb of the subject. In particular, this guidance reference image A comprises a lateral view of the skin of: part of the thigh, the knee, and part of the lower leg.

**Fig. 3B** is a schematic representation of a first image B captured in the second capturing step 200 of fig. 2B. The first image B depicts a portion of the subject's lower limb that at least partially overlaps with the same portion of lower limb in the previously captured guidance reference image A of fig. 2A. The first image B also comprises a calibration reference 2 adhered on the subject's skin.

A similarity metric is calculated between the guidance reference image A and the first image. The similarity metric is then used to generate an overlay that is displayed during one or more capturing steps 400 of capturing additional images of the set of monitoring images ("subsequent images").

**Fig. 3C** is a schematic representation of a subsequent image C, in which the guidance reference image A of fig. 2A is displayed as an overlay, e.g. during a live image preview.

In this example, overlay comprises the edges of the lower limb and the skin alteration. This overlay can be a mask generated based on boundaries of the lower limb and the skin alteration (e.g. obtained using edge detection algorithms) and on the similarity metric (e.g. a distance or percentage of overlap between detected edges). The mask can be overlapped or blended to a live preview of the subsequent image C.

The overlay may include visual indicia (not shown in fig. 3C) indicating whether the similarity metric falls below or exceeds a predefined threshold. Such indicia may include visual cues such as green or light-colored indicators, or text suggesting that the current moment is optimal for capturing the subsequent image C. This allows to provide feedback to the user about whether the current capturing conditions are approaching or deviating (e.g. in response to the user moving the device 1) from the intended capturing conditions.

The visual indicia may be further be based on positional data received from sensors (e.g. from an accelerometer, or a gyroscope integrated in the device 1), to guide the user on how to adjust the position or orientation of the device 1, until the similarity metric exceeds the threshold.

The device 1 for capturing the guidance reference image A, the first image B and one or more of the subsequent images C may be the same device 1 (as in the examples of fig. 2A and fig. 2B); however, different devices 1 can be used.

**Fig. 4** is a schematic representation of a calibration reference 2.

The calibration reference 2 comprises a geometric reference 21 and/or a color reference 22, respectively comprising a plurality of two-dimensional markers and color markers. In this example, the two-dimensional markers and color markers are arranged in a grid pattern, forming a board of alternated markers. However, the calibration reference 2 may comprise a different arrangement of the two-dimensional markers and color markers.

The two-dimensional markers can be Aruco markers, as shown in fig. 4, However, different two-dimensional markers (e.g. two-dimensional barcodes, or QR codes) or one-dimensional markers (e.g., rulers) can also be used.

The geometric reference 21 in the first image B can be used to adjust geometric parameters of the guidance reference image A and/or of the subsequent image C, to match the corresponding geometric parameters of the first image B (geometric calibration).

This allows to improve accuracy of geometric measurements done on the first image B and/or of the subsequent image C.

Geometric measurements may comprise for instance a wound length, width, depth. Geometric measurements may also comprise a surface/perimeter ratio of a wound.

In the example of fig. 4, the calibration reference 2 comprises a color reference 22 that, in this case, comprises five color markers that are repeated and alternated with the two-dimensional markers, forming a board. For example, the color markers may comprise one or more of a red marker, a yellow marker, a brown marker, a black marker, and grey marker. However, different color markers can be selected e.g. based on the skin process that is monitored over time.

The color reference 22 in the first image B can be used to adjust color parameters of the guidance reference image A and/or of the subsequent image C, to match the corresponding color parameters of the first image B (color calibration).

This allows to improve accuracy of color measurements done on the first image B and/or of the subsequent image C.

Color measurements may comprise for instance a number of pixels classified in a predefined tissue class (e.g. fibrous tissue, necrotic tissue, blood, exudate or epithelial tissue) based on their color.

The geometric and/or color measurements may be repeated on each image of the set of monitoring images to monitor changes of the skin over time.

It should be understood that the present invention is not limited to the described embodiments and that variations can be applied without going outside of the scope of the claims.

## Claims

1. Computer-implemented method for monitoring skin changes over time of the skin of a subject, the method comprising the steps of:
- capturing (100) a guidance reference image (A) comprising a portion of the skin of the subject;
- capturing (200) a first image (B) of a set of monitoring images comprising the portion of the skin of the subject and further comprising a calibration reference adhered to the skin;
- calculating (300) a similarity metric between the guidance reference image (A) and the first image (B) of the set;
wherein, during the capturing (400) of subsequent images (C) of the set of monitoring images the guidance reference image (A) is displayed as an overlay based on the similarity metric thereby providing guidance to a user during capturing (400).

2. The method according to claim 1, wherein the overlay of the guidance reference image (A) comprises:
- applying a transparency coefficient to the guidance reference image; and/or
- generating a mask of the guidance reference image (A) using an edge detection algorithm.

3. The method according to claim 1 or claim 2, further comprising a step of calibrating the guidance reference image (A) based on the calibration reference (2).

4. The method according to any of the preceding claims, wherein the calibration reference (2) comprises a geometric reference (21).

5. The method according to claim 4, wherein the geometric reference (21) comprises a one-dimensional marker and/or a two-dimensional marker.

6. The method according to claim 5, wherein the two-dimensional marker comprises one or more of the group of a Aruco marker, a two-dimensional barcode, a QR code.

7. The method according to any one of claims 1 to 3, wherein the calibration reference (2) comprises a color reference (22).

8. The method according to claim 7, wherein the color reference (22) comprises one or more of the group of a red marker, a yellow marker, a black marker, a brown marker, a grey marker.

9. The method according to any of the preceding claims, wherein the calibration reference (2) comprises at least one color reference (22) and at least one geometric reference (21) arranged in a grid pattern.

10. The method according to any of the preceding claims, wherein the similarity metric is based on one of the group of a correlation; contour matching; features matching.

11. The method according to any of the preceding claims, the method further comprising the steps of:
- segmenting the subsequent images of the set of monitoring images thereby isolating a region of interest comprising a skin alteration from a background;
- classifying pixels of the isolated region of interest into a class of a set of predefined tissue classes based on a color of the pixel;
wherein the predefined tissue classes comprise at least one of: fibrous tissue, necrotic tissue, blood, serous exudate or epithelial tissue.

12. The method according to claim 11, wherein the region of interest comprises a skin lesion, the skin lesion being one of the group of: a wound; a scar; an ulcer; and/or a burn.

13. Skin monitoring device (1) for monitoring skin changes over time of the skin of a subject comprising means configured to perform the method according to any of the preceding claims.

14. A computer readable storage medium comprising instructions which, when executed by a computer, cause the computer to carry out the steps of the method according to any one claim 1 to claim 12.

15. Computer program comprising program code instructions which, when executed by a computer, cause the computer to carry out the method according to any one claim 1 to claim 12.
